# NEW EUROPEAN PATENT SPECIFICATION

(11) **EP 2 545 777 B2**
(45) Date of publication and mention of the opposition decision: **18.01.2023**
(45) Mention of the grant of the patent: 11.03.2020
(21) Application number: 12188400.1
(22) Date of filing: 15.08.2008
(51) Int. Cl.: A01N 43/80, A01N 47/12, A01N 47/18, A01N 53/00, A01P 7/00, A61K 31/42

(54) **Use of an isoxazoline composition as antiparasitic**
Isoxazolinverbindungen und ihre Verwendung als Antiparasitika
Compostions d'isoxazoline et leur utilisation en tant qu'antiparasitaires

(30) Priority: 17.08.2007 US 956448 P; 17.08.2007 EP 07016152; 21.12.2007 EP 07150309; 14.07.2008 US 80444
(43) Date of publication of application: 16.01.2013
(62) Divisional of application: 08803041.6
(73) Proprietor: Intervet International B.V., 5831 AN Boxmeer (NL)
(72) Inventor: Williams, Heike, 55270 Schwabenheim (DE); Heckeroth, Anja Regina, 55270 Schwabenheim (DE); Lutz, Jürgen, 55270 Schwabenheim (DE); Mertens, Christina, 5831 AN Boxmeer (NL); Zoller, Hartmut, 55270 Schwabenheim (DE); Mita, Takeshi, Chiba 722-1 (JP)
(74) Representative: Intervet International B.V.

(56) References cited:
- WO-A1-98/42191
- WO-A1-2007/079162
- WO-A1-2009/003075
- WO-A1-2009/003075
- WO-A2-2007/075459
- WO-A2-2009/024541
- US-A1- 2007 066 617
- THOMAS ET AL.: "Field efficacy and safety of fluralaner solution for administration in drinking water for the treatment of poultry red mite (Dermanyssus gallinae) infestations in commercial flocks in Europe", PARASITES & VECTORS, vol. 10.457, 2017, pages 1-9,
- GUILLAUME ET AL.: "METHODOLOGIE DE SUIVI DES POPULATIONS DE DERMANYSSUS GALLINAE EN ELEVAGE DE PONDEUSE AVEC PARCOURS EXTERIEUR", CINQUIÈMES JOURNÉES DE LA RECHERCHE AVICOLE , TOURS, vol. 26, 27 March 2003 (2003-03-27)
- "LABORATORY ANIMAL MEDICINE", 2015, article WHARY ET AL.: "chapter 3 Biology and Diseases of Mice", pages: 43 - 149
- "Canine Parasites and Parasitic Diseases", 10 November 2018, article SAARI ET AL.: "chapter 9 Arachnida", pages: 187 - 228
- DHILLON ET AL.: "Basic Pharmacokinetics", CLINICAL PHARMACOKINETICS, March 2006 (2006-03-01), London
- J. CORBA et al.: "Efficacy of injectable moxidectin against mixed (Psoroptes ovis and Sarcoptes scabiei var. ovis) mange infestation in sheep", Veterinary Parasitology, vol. 56, 1 February 1995 (1995-02-01), pages 339-344,
- W. H. MILLER et al.: "Treatment of canine scabies with milbemycin oxime", Can. Vet. J., vol. 37, April 1996 (1996-04), pages 219-221,
- M. PARADIS et al.: "Topical (pour-on) ivermectin in the treatmentof canine scabies", Can. Vet. J., vol. 38, June 1997 (1997-06), pages 379-382,
- C. CHAUVE: "The poultry red mite dermanyssus gallinae (De Geer, 1778): current situation and future prospects for control", Veterinary Parasitology, vol. 79, 16 November 1998 (1998-11-16), pages 239-245,
- M. JACOBSON et al.: "The efficacy of simplified eradication strategies against sarcoptic mange mite infections in swine herds monitored by an ELISA", Veterinary Parasitology, vol. 81, 1 February 1999 (1999-02-01), pages 249-258,
- R. WALL et al.: Veterinary ectoparasites - Biology, Pathology & Control, June 2001 (2001-06),
- L. L. VIZCAINO et al.: "EXPERIMENTAL IVERMECTIN TREATMENT OF SARCOPTIC MANGE AND ESTABLISHMENT OF A MANGE-FREE POPULATION OFSPANISH IBEX", Journal of Wildlife Diseases, vol. 37, no. 4, November 2001 (2001-11), pages 775-785,
- J C E JENSEN et al.: "Elimination of Mange Mites Sarcoptes scabiei var. suis from Two Naturally Infested Danish Sow Herds Using a Single Injection Regime with Doramectin", Acta Vet. Scand., vol. 43, no. 2, 30 June 2002 (2002-06-30), pages 75-84,
- N. CHAILLEUX et al.: "Efficacy of selamectin in the treatment of naturally acquired cheyletiellosis in cats", Can. Vet. J., vol. 43, October 2002 (2002-10), pages 767-770,
- R. S. MUELLER et al.: "Efficacy of selamectin in the treatment of canine cheyletiellosis", The Veterinary Record, vol. 151, 28 December 2002 (2002-12-28), page 773,
- O. HANSEN et al.: "Efficacy of a formulation Containing Imidacloprid and Moxidectin Against Naturally Acquired Ear Mite Infestations (Psoroptes cuniculi) in Rabbits", Intern J Appl Res Vet Med, vol. 3, no. 4, 2005, pages 281-286,
- V. JEKL et al.: "Demodicosis in nine prairie dogs {Cynomys ludovicianus)", European Society of Veterinary Dermatology, vol. 17, September 2006 (2006-09), pages 280-283,
- J . J. FOURIE et al.: "Comparative Efficacy and Safety of Two Treatment Regimens with a Topically Applied Combination of Imidaclopri'd and Moxidectin (Advocate®) against Generalised Demodicosis in Dogs", Parasitol Res, vol. 105, 28 June 2009 (2009-06-28), pages S115-S124,
- Y. OZOE et al.: "The antiparasitic isoxazoline Al443 is a potent blocker of insect ligand-gated chloride channels", Biochemical and Biophysical Research Communications, vol. 391, 26 November 2009 (2009-11-26), pages 744-749,
- "Recommended INN: List 69", WHO Drug Information, vol. 27, no. 1, April 2013 (2013-04), page 59,
- E. THOMAS et al.: "Field efficacy and safety of fluralaner solution for administration in drinking water for the treatment of poultry red mite (Dermanyssus gallinae) infestations in commercial flocks in Europe", Parasites & Vectors, vol. 10, no. 457, 9 October 2017 (2017-10-09), pages 1-10,
- Courrier du titulaire du 21/11/2018
- Annex 1 - Summary of Exzolt products characteristics
- EMA-CVMP assessment report for Exzolt (2017)

## Description

### FIELD OF THE INVENTION

This invention relates to methods for controlling parasitic infestations of animals and their environments, and, more particularly, to methods using isoxazolines to control parasites in their environments.

### BACKGROUND OF THE INVENTION

A number of pests and parasites are known to infest warm-blooded animals. These pests and parasites can be great nuisances to both the animals and their owners. For example, virtually all companion and livestock animals can be affected by ectoparasites, such as ticks, mites, lice, and fleas. Ectoparasites tend to irritate the animals, and also can cause clinical disease and adverse sub-clinical conditions, either by themselves or by carrying vector-transmitted pathogens. To date, various treatments have been developed to control ectoparasites on warm-blooded animals. US2007/066617 discloses the use of isoxazoline-substituted benzamine compound for controlling domestic animal pests. Nevertheless, a need continues to exists for compositions (and methods for their use) that are bioavailable, can provide contact or systemic activity, are potently efficacious, have a quick onset of activity, have a long duration of activity, and/or are safe to the animal recipients and/or their human owners. This invention addresses this need.

### SUMMARY OF THE INVENTION

The scope of the invention is defined by the claims. Any references in the description to methods of treatments refer to the compounds for use in a method for treatment of the animal body by therapy. This invention is directed, to an isoxazoline compound, a salt of the isoxazoline, or a solvate of the isoxazoline or salt; the isoxazoline corresponds in structure to Formula(I): 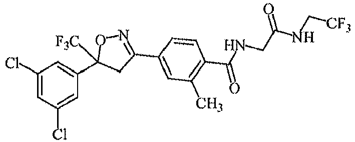 for use in effectively controlling an ectoparasite infestation of an animal, wherein the ectoparasites are mites and the isoxazoline compound is administered multiple times for a single treatment to the animal and such treatment controls such mites in the environment that is occupied by the animal.

Further benefits of Applicants' invention will be apparent to one skilled in the art from reading this specification.

### DETAILED DESCRIPTION OF PREFERRED EMBODIMENTS

### I. The isoxazoline

The isoxazoline used in of Formula (I) in accordance with this invention is a compound:

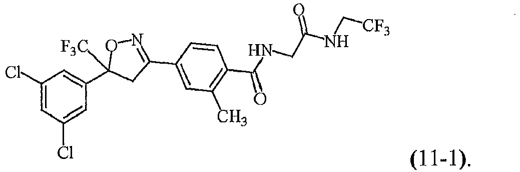

The chemical name for this isoxazoline is 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide. It can be found in, for example, CAS RN [864731-61-3].

### I. Isomers

The isoxazoline used in this invention generally can have two or more conformational structures. At minimum, the isoxazoline comprises a chiral (or asymmetric) carbon at the 5-position of the isoxazoline ring. In some embodiments, for example, the chiral carbon has a left-handed (or "S" or "sinister") configuration. An example of such a compound is:

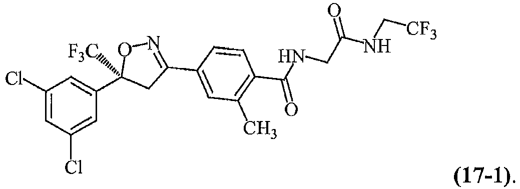

In other embodiments, the chiral carbon has a right-handed (or "R" or "rectus") configuration.

An example of such a compound is:

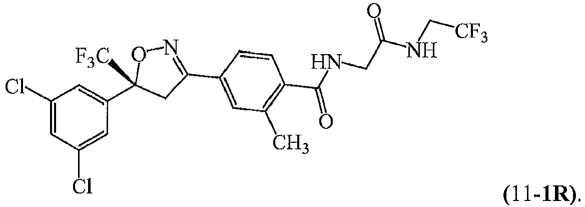

A specific isomer often can be isolated from the corresponding racemic mixture (or a salt thereof) using, for example, chiral high performance liquid chromatography (HPLC) techniques. Such a technique is illustrated in **Example 7** below for isolating the R and S enantiomers of racemic Compound **11-1**. In some instances when an isomer is difficult to separate, a more-easily-isolatable derivative of the isomer is isolated from the corresponding derivative racemic mixture (or a salt thereof), and then converted to the isomer. Alternatively, a specific isomer often can be directly synthesized from, for example, an optically pure starting material.

In some embodiments, the ratio of one enantiomer (*e.g.*, Compound 17-1) to another enantiomer (*e.g.*, Compound **11-1**R) in the pharmaceutical composition used with this invention is greater than 1:1. In some instances, for example, the ratio is greater than about 70:30, greater than about 85:15, greater than about 90:10, greater than about 95:5, greater than about 98:2, or greater than about 99:1.

In some embodiments, the concentration of one enantiomer (*e.g.*, Compound 17-1) in the composition (or, more typically, a precursor composition) is greater than about 50% (by weight). In some such embodiments, for example, the concentration is greater than about 70% (by weight), greater than about 85% (by weight), greater than about 90% (by weight), greater than about 95% (by weight), greater than about 98% (by weight), greater than about 99% (by weight), or greater than about 99.5% (by weight).

Unless otherwise stated, a isoxazoline structure that does not indicate a particular conformation is intended to encompass compositions of all the possible conformational isomers of the isoxazoline, as well as compositions comprising fewer than all (*e.g.*, just one of) the possible conformational isomers.

### J. Salts of the isoxazolines

As noted above, many isoxazolines used with this invention may be in the form of a salt. A salt may be advantageous due to one or more of its physical properties, such as pharmaceutical stability in differing temperatures and humidities; crystalline properties; and/or a desirable solubility in water, oil, or other solvents. Acid and base salts typically can be formed by, for example, mixing a compound with an acid or base, respectively, using various known methods in the art. In general, when the salt is intended to be administered *in vivo* (*i.e.*, to an animal) for a therapeutic benefit, the salt preferably is pharmaceutically acceptable.

In some instances, a base addition salt of the isoxazoline of **Formula (I)** can be prepared by reacting the isoxazoline with an approximately stoichiometric amount of an inorganic or organic base, typically a strong inorganic or organic base. Examples of base addition salts may include, for example, metallic salts, and organic salts. Metallic salts, in particular, include alkali metal (group la, *e.g.*, lithium, sodium, or potassium) salts, alkaline earth metal (group IIa, *e.g.*, barium, calcium, and magnesium) salts, heavy metal (*e.g.*, zinc and iron) salts, and other physiologically acceptable metal salts. Such salts may be made from calcium, lithium, magnesium, potassium, sodium, and zinc. For example, a free acid isoxazoline may be mixed with sodium hydroxide to form such a base addition salt.

In some instances, an acid addition salt of the isoxazoline of **Formula (I)** can be prepared by reacting the isoxazoline with an approximately stoichiometric amount of an inorganic or organic acid. Examples of contemplated inorganic acids for making pharmaceutically acceptable salts include hydrochloric, hydrobromic, hydroiodic, nitric, carbonic, sulfuric, and phosphoric acid. Examples of often suitable organic acids for making pharmaceutically acceptable salts generally include, for example, aliphatic, cycloaliphatic, aromatic, araliphatic, heterocyclic, carboxylic, and sulfonic classes of organic acids. Specific examples of organic acids include cholic, sorbic, lauric, acetic, trifluoroacetic, formic, propionic, succinic, glycolic, gluconic, digluconic, lactic, malic, tartaric acid, citric, ascorbic, glucuronic, maleic, fumaric, pyruvic, aspartic, glutamic, aryl carboxylic acid (*e.g.*, benzoic), anthranilic acid, mesylic, stearic, salicylic, p-hydroxybenzoic, phenylacetic, mandelic, embonic (pamoic), alkylsulfonic (*e.g.*, ethanesulfonic), arylsulfonic (*e.g.*, benzenesulfonic), pantothenic, 2-hydroxyethanesulfonic, sulfanilic, cyclohexylaminosulfonic, β-hydroxybutyric, galactaric, galacturonic, adipic, alginic, butyric, camphoric, camphorsulfonic, cyclopentanepropionic, dodecylsulfic, glycoheptanoic, glycerophosphic, heptanoic, hexanoic, nicotinic, 2-naphthalesulfonic, oxalic, palmoic, pectinic, 3-phenylpropionic, picric, pivalic, thiocyanic, tosylic, and undecanoic acid.

In some instances, an organic salt of the isoxazoline of **Formula (I)** may be made by, for example, quaternizing a basic nitrogen-containing group on the isoxazoline with an agent such as a C₁-C₆-alkyl halide (*e.g.*, methyl, ethyl, propyl, and butyl chlorides, bromides, or iodide), dialkyl sulfate (*e.g.*, dimethyl, diethyl, dibuytl, or diamyl sulfate), long chain halide (*e.g.*, decyl, lauryl, myristyl, and stearyl chlorides, bromides, and iodide), arylalkyl halide (*e.g.*, benzyl and phenethyl bromide), and the like.

It should be understood that the counterion of an acid or base salt may, in some instances, be optically active (*e.g*., D-lactate and L-lysine salts) or racemic (*e.g*., DL-tartrate and DL-arginine salts).

### K. Solvates of the isoxazoline

In some instances, the isoxazoline of **Formula (I)** is in the form of stable complexes with solvent molecules that remain intact after the non-complexed solvent molecules are removed from the compounds. These complexes generally are referred to as "solvates." In some instances, the solvate will be capable of isolation, for example when one or more solvent molecules are incorporated into the crystal lattice of the crystalline solid. A "solvate" encompasses both solution-phase and isolatable solvates. Examples of suitable solvates include ethanolates, methanolates, and the like. A "hydrate" is a solvate wherein the solvent molecule is water. A solvate intended to be used *in vivo* preferably is pharmaceutically acceptable.

### II. Preparation of the isoxazoline of Formula (I)

Methods for preparing isoxazolines are known in the art. Methods for preparing isoxazoline of **Formula (I)**, for example, have been discussed in US Patent Publ. No. US2007/0066617. For example, US Patent Publ. No. US2007/0066617 discusses the preparation of Compound **11-1** at Example 21 on page 72.

### III. Treatment Methods Using a Composition of this Invention

The isoxazoline of Formula (I) generally is used to control mites on animals, and, in turn, diseases directly caused by such ectoparasites and/or diseases caused by pathogens carried by such ectoparasites. It is contemplated that the composition may be used to treat a range of animals, especially warm-blooded animals. Such warmblooded animals include, for example, mammals. Mammals include, for example, humans. Other mammals include, for example, farm or livestock mammals (e.g., swine, bovines, sheep, goats, etc.), laboratory mammals (e.g., mice, rats, jirds, etc.), companion mammals (e.g., dogs, cats, equines, etc.), fur-bearing animals (e.g., minks, foxes, chinchillas, rabbits, etc.), and wild and zoo mammals (e.g., buffalo, deer, etc.). In some embodiments, the compositions are used to treat canines (e.g., dogs, such as, for example, pure-bred and/or mongrel companion dogs, show dogs, working dogs, herding dogs, hunting dogs, guard dogs, police dogs, racing dogs, and/or laboratory dogs). In other embodiments, the compositions are used to treat felines (e.g., domestic cats). It is contemplated that the compositions also are suitable to treat non-mammals, such as birds (e.g., turkeys, chickens, geese, ducks, parrots, etc.). It is also contemplated that such compositions may be useful to treat cold-blooded animals as well, such as, for example, fish (e.g., salmon, trout, koi, etc.).

It has been discovered in accordance with this invention that the isoxazoline of **Formula (I)** is generally of particular value for controlling mites that are injurious to, or spread or act as vectors of diseases in, warm-blooded animals. The isoxazolines are generally beneficial for controlling various lifecycle stages of parasites, including egg, nymph, larvae, juvenile, and adult stages Mites include:
i. *Mesostigmata* spp., such as mesostigmatids, which include chicken mites (*Dermanyssus gallinae*).
ii. *Astigmata* spp., such as itch or scab mites, which include *Sarcoptidae* spp. (*e.g.*, *Sarcoptes scabiei*); and mange mites, which include *Psoroptidae* spp. (*e.g*., *Chorioptes bovis* and *Psoroptes ovis*).
iii. *Prostigmata* spp, such as chiggers, which include *Trombiculidae* spp. (*e.g.*, North American chiggers, *Trombicula alfreddugesi).*
iv. Demodex.
An "infestation" refers to the presence of parasites in numbers that pose a risk of nuisance or harm to humans or animals. The presence can be in the environment (*e.g*., in animal bedding), on the skin or fur of an animal, etc. Unless otherwise stated, when the infestation is within an animal (*e.g*., in the blood or other internal tissues), the term infestation is intended to be synonymous with the term, "infection," as that term is generally understood in the art.

The phrase "control of ectoparasite infestation" means to reduce or eradicate parasite numbers in and/or on an animal, and/or to partially or completely inhibit the development of parasite infestation in and/or on an animal. This may be achieved by, for example, killing, repelling, expelling, incapacitating, deterring, eliminating, alleviating, or minimizing the parasite. The control of ectoparasites can be insecticidal and/or acaricidal. The effect of the isoxazoline can be, for example, ovicidal, larvicidal, nymphicidal, adulticidal, or a combination thereof. In addition, the effect can manifest itself directly by killing the parasites either immediately or after some time has elapsed (*e.g*., when molting occurs or by destroying eggs). The effect alternatively (or additionally) can manifest itself indirectly by, for example, reducing the number of eggs laid and/or the hatch rate.

In general, an amount of a isoxazoline that is sufficient to "control" or be "effective" against a target parasite is an amount that is sufficient to reduce or eradicate parasite numbers in and/or on an animal, and/or to partially or completely inhibit the development of parasite infestation in and/or on an animal. When the isoxazoline is administered systemically, an effective amount generally constitutes an amount that results in tissue and/or blood concentrations generally toxic when ingested by a target parasite.

One of ordinary skill in the art typically can determine an "effective" dose by, for example, observing or detecting changes in a clinical condition or behavior of a host animal, as well as by observing or detecting relative changes in parasite numbers after such treatment. In general, a dose is considered effective for controlling a target parasite when the dose is sufficient to cause an existing or potential target parasite count to be reduced by at least about 5%. In some such instances, for example, the dose is considered effective when the dose is sufficient to cause an existing or potential parasite count to be reduced by at least about 10% (or at least about 30%, at least about 50%, at least about 60%, at least about 75%, at least about 90%, at least about 95%, or at least about 99%).

The optimum dosage generally depends on multiple factors, including, for example, the particular isoxazoline; the identity of any other active ingredient(s) being administered to the animal recipient; the route of administration; the type and severity of the target condition and pathogen; the type (*e.g*., species and breed), age, size, sex, diet, activity, and condition of the intended animal recipient; and pharmacological considerations, such as the activity, efficacy, pharmacokinetic, and toxicology profiles of the isoxazoline and other active ingredient(s) being administered to the recipient animal. To the extent multiple active ingredients are administered for combined effects on a target parasite or condition, the amount of each ingredient that constitutes an "effective amount" is an amount that, when combined with the other active ingredients, causes the desired effect.

The isoxazoline of **Formula (I)** is administered multiple times for a single treatment. Such multiple-dosage treatments are contemplated to include multiple doses per day for one or more days, daily doses for multiple days, and/or doses administered two or more days apart. The durations over which the isoxazoline of **Formula (I)** tend to be effective against various ectoparasites by systemic administration is surprising. This is particularly true, given that such long activities may, in many instances, be obtained using low doses that are non-toxic to the animal recipients without requiring the use of a controlled-release means. Without being limited to any particular theory, it is hypothesized that this long duration of activity stems from the isoxazolines having particularly high toxicity when ingested by the target parasite.

For many animal recipients, the isoxazoline dose and formulation are chosen to maintain a isoxazoline serum level of at least about 1 ng/ml (*e.g*., 1 to 50 ng/ml). In general, the amount of isoxazoline administered to the animal recipient is from about 0.001 to about 200 mg/kg body weight. In some embodiments, for example, from about 0.01 to about 200 mg/kg body weight is administered. In other embodiments, for example, from about 0.001 to about 100 mg/kg body weight is administered. In some such embodiments, for example, from about 0.01 to about 100 mg/kg body weight is administered. In other such embodiments, from about 1 to about 30 mg/kg body weight is administered. Greater dosages tend to provide for greater duration of activity.

It is contemplated that the duration of activity of a isoxazoline can be extended even further (or made more consistent) by using a controlled-release formulation or dosage form. For example, the isoxazoline can be administered in microspheres, granules, or implants (*e.g*., a subcutaneous implant) that release the isoxazoline by, example, diffusion and/or erosion. Use of such a dosage form containing from about 1 and about 50 mg/kg body weight (or from about 10 to about 30 mg/kg body weight, such as about 20 mg/kg of body weight) of the isoxazoline may allow for consistent activity lasting over several months or longer (*e.g*., a year).

In some embodiments of this invention, the isoxazoline of **Formula (I)** is administered to treat parasitoses of an animal (or make a medicament to treat parasitoses of an animal). The term "parasitoses" includes pathologic conditions and diseases associated with or caused by one or more ectoparasites directly. The phrase "treatment of parasitoses" means to partially or completely inhibit the development of parasitoses of an animal susceptible to parasitoses, reduce or completely eliminate the symptoms of parasitoses of an animal having parasitoses, and/or partially or completely cure parasitoses of an animal having parasitoses. In general, the treatment of parasitoses is achieved by administering the isoxazoline of **Formula (I)** to control an ectoparasite infestation.

This invention also relates to treatment methods wherein at least an ancillary goal of controlling ectoparasites in and/or on an animal is to control an ectoparasitic infestation in an environment that is occupied (periodically or continuously) by the animal, wherein the ectoparasites are mites. In some such embodiments, for example, the animal is a companion animal (e.g., a cat or dog). The environment may be, for example, a house or other shelter; a room; a pen, a stall, or other confinement means; bedding; etc.

The terms "administer" and "administration" refer to the delivery of the isoxazoline of **Formula (I)**, salt of the isoxazoline, solvate of the isoxazoline or salt, or prodrug of the isoxazoline. In some embodiments of this invention, systemic administration is desirable. "Systemic administration" is an administration at a site remote from a site wherein at least a portion of the target parasites reside. With systemic administration, at least a portion of the isoxazoline reaches the target parasite via the animal recipient's bloodstream, other body fluids (lymph fluids), and/or tissues (*e.g*., skin or fat tissue). Typically, the parasite ingests the isoxazoline along with the animal recipient's blood, other body fluids, and/or tissue. Systemic administration may be achieved in several forms.

In some embodiments, the isoxazoline composition is systemically administered via an oral route in a unit dosage form, such as, for example, a soft or hard capsule, a pill, a powder, granules, a tablet (*e.g*., a chewable tablet), a paste, a solution, a suspension (aqueous or non-aqueous), an emulsion (oil-in-water or water-in-oil), an elixir, a syrup, a bolus, a drench, or via the animal recipient's feed or drinking water. When the composition is administered via an animal's feed, it may, for example, be fed as a discrete feed or as a chewable treat. Alternatively (or additionally), it may, for example, be intimately dispersed in the animal recipient's regular feed, used as a top dressing, or in the form of pellets or liquid that is added to the finished feed. When the composition is administered as a feed additive, it may be convenient to prepare a "premix" in which the composition is dispersed in a liquid or solid carrier. This "premix" is, in turn, dispersed in the animal's feed using, for example, a conventional mixer. When the composition is administered in the animal recipient's drinking water or as a drench, it may be convenient to use a solution or suspension formulation. This formulation can be, for example, a concentrated suspension that is mixed with water or a dry preparation that is mixed and suspended in the water. In both instances, it is preferable to have the isoxazoline in a finely-pulverized form.

The isoxazoline composition alternatively (or additionally) may be systemically administered topically using a transdermal formulation (*i.e*., a formulation that passes through the skin). Alternatively (or additionally), the composition may be systemically administered topically via the mucosa. Typical formulations for transdermal and mucosal administration include, for example, pour-ons, spot-ons, dips, sprays, mousses, shampoos, powders, gels, hydrogels, lotions, solutions, creams, ointments, dusting powders, dressings, foams, films, skin patches, limb bands, collars, ear tags, wafers, sponges, fibers, bandages, and microemulsions. The pour-on or spot-on methods, for example, comprise applying the isoxazoline composition to a specific location of the skin or coat, such as on the neck or backbone of the animal. This may be achieved by, for example, applying a swab or drop of the pour-on or spot-on formulation to a relatively small area of the recipient animal's skin or coat (*i.e*., generally no greater than about 10% of the animal recipient's skin or coat). In some embodiments, the isoxazoline is dispersed from the application site to wide areas of the fur due to the spreading nature of the components in the formulation and the animal's movements while, in parallel, being absorbed through the skin and distributed via the animal recipient's fluids and/or tissues.

The isoxazoline composition alternatively (or additionally) may be systemically administered parenterally, such as via intramuscular injection, intravenous injection, subcutaneous injection, implant (*e.g*., subcutaneous implant), infusion, bolus, etc. In some such embodiments, the parenteral dosage form provides the animal recipient with from about 0.01 to about 200 mg/kg body weight of the isoxazoline.

Other contemplated modes of administration include, for example, rectal, vaginal, and via inhalation (*e.g*., via a mist or aerosol).

### IV. Pharmaceutical compositions

Also disclosed are pharmaceutical compositions (or medicaments) comprising the isoxazoline of **Formula (I)**, salt of the isoxazoline, solvate of the isoxazoline or salt, or prodrug of the isoxazoline. The compositions also may (and generally will) comprise one or more pharmaceutically-acceptable excipients.

Pharmaceutical compositions of the present invention may be manufactured by, for example, processes known in the art. These processes include, for example, a variety of known mixing, dissolving, granulating, emulsifying, encapsulating, entrapping, and lyophilizing processes. Optimal formulation depends on, for example, the route of administration.

Solid dosage forms, for example, may be prepared by, for example, intimately and uniformly mixing the isoxazoline with fillers, binders, lubricants, glidants, disintegrants, flavoring agents (*e.g*., sweeteners), buffers, preservatives, pharmaceutical-grade dyes or pigments, and controlled release agents.

Oral dosage forms other than solids may be prepared by mixing the isoxazoline with, for example, one or more solvents, viscosity-enhancing agents, surfactants, preservatives, stabilizers, resins, fillers, binders, lubricants, glidants, disintegrants, co-solvents, sweeteners, flavorings, perfuming agents, buffers, suspending agents, and pharmaceutical-grade dyes or pigments.

Contemplated binders include, for example, gelatin, acacia, and carboxymethyl cellulose.

Contemplated lubricants include, for example, magnesium stearate, stearic acid, and talc.

Contemplated disintegrants include, for example, corn starch, alginic acid, sodium carboxymethylcellulose, and sodium croscarmellose.

Contemplated buffers include, for example, sodium citrate, and magnesium and calcium carbonate and bicarbonate.

Contemplated solvents include, for example, water, petroleum, animal oils, vegetable oils, mineral oil, and synthetic oil. Physiological saline solution or glycols (*e.g*., ethylene glycol, propylene glycol, or polyethylene glycol) also may be included. The solvent preferably has sufficient chemical properties and quantity to keep the isoxazoline solubilized at temperatures in which the composition is stored and used.

Contemplated viscosity-enhancing agents include, for example, polyethylene, methylcellulose, sodium carboxymethylcellulose, hydroxypropylmethylcellulose, hydroxypropylcellulose, sodium alginate, carbomer, povidone, acacia, guar gum, xanthan gum, tragacanth, methylcellulose, carbomer, xanthan gum, guar gum, povidone, sodium carboxymethylcellulose, magnesium aluminum silicate, carboxyvinyl polymers, carrageenan, hydroxyethyl cellulose, laponite, water-soluble salts of cellulose ethers, natural gums, colloidal magnesium aluminum silicateor finely divided silica, homopolymers of acrylic acid crosslinked with an alkyl ether of pentaerythritol or an alkyl ether of sucrose, and carbomers.

Contemplated surfactants include, for example, polyoxyethylene sorbitan fatty acid esters; polyoxyethylene monoalkyl ethers; sucrose monoesters; lanolin esters and ethers; alkyl sulfate salts; and sodium, potassium, and ammonium salts of fatty acids.

Contemplated preservatives include, for example, phenol, alkyl esters of parahydroxybenzoic acid (*e.g*., methyl *p*-hydroxybenzoate (or "methylparaben") and propyl *p*-hydroxybenzoate (or "propylparaben")), sorbic acid, o-phenylphenol benzoic acid and the salts thereof, chlorobutanol, benzyl alcohol, thimerosal, phenylmercuric acetate and nitrate, nitromersol, benzalkonium chloride, and cetylpyridinium chloride.

Contemplated stabilizers include, for example, chelating agents and antioxidants.

Solid dosage forms also may comprise, for example, one or more excipients to control the release of the isoxazoline. For example, it is contemplated that the isoxazoline may be dispersed in, for example, hydroxypropylmethyl cellulose. Some oral dosage forms (*e.g*., tablets and pills) also may be prepared with enteric coatings.

Topical administration may be achieved using, for example, a concentrated solution, suspension (aqueous or non-aqueous), emulsion (water-in-oil or oil-in-water), or microemulsion comprising a isoxazoline dissolved, suspended, or emulgated in a pharmaceutically-acceptable liquid vehicle. In such embodiments, a crystallization inhibitor optionally may generally be present.

When a liquid formulation is used topically on skin, it can be administered by, for example, pouring on, spreading, rubbing, atomizing, spraying, dipping, bathing, or washing. A pour-on or spot-on formulation, for example, can be poured or atomized onto a limited spot on the skin (typically no greater than about 10% of the skin). In some such embodiments, the formulation allows or facilitates the isoxazoline to penetrate the skin and act on other parts body (*e.g*., the entire body). Such a pour-on or spot-on formulation can be prepared by dissolving, suspending, or emulsifying the isoxazoline in a suitable skin-fitted solvent or solvent mixture. Other excipients may be included as well, such as, for example, a surfactant, colorant, antioxidant, stabilizer, adhesive, etc. Contemplated solvents include, for example, water, alkanol, glycol, polyethylene glycol, polypropylene glycol, glycerin, benzyl alcohol, phenylethanol, phenoxyethanol, ethyl acetate, butyl acetate, benzyl benzoate, dipropylene glycol monomethyl ether, diethylene glycol monobutyl ether, acetone, methyl ethyl ketone, aromatic and/or aliphatic hydrocarbons, vegetable or synthetic oil, DMF, liquid paraffin, silicone, dimethylacetamide, N-methylpyrrolidone, or 2,2-dimethyl-4-oxy-methylene-1,3-dioxolane.

In some embodiments, a topical formulation (particularly a pour-on or spot-on formulation) comprises a carrier that promotes the absorption or penetration of the isoxazoline through the skin into the blood stream, other bodily fluids (lymph), and/or body tissue (fat tissue). Contemplated examples of dermal penetration enhancers include, for example, dimethylsulfoxide, isopropyl myristate, dipropylene glycol pelargonate, silicone oil, aliphatic esters, triglycerides, and fatty alcohols.

Topical formulations also (or alternatively) may comprise, for example, one or more spreading agents. These substances act as carriers that assist in distributing an active ingredient over the animal recipient's coat or skin. They may include, for example, isopropyl myristate, dipropylene glycol pelargonate, silicone oils, fatty acid esters, triglycerides, and/or fatty alcohols. Various spreading oil/solvent combinations also may be suitable, such as, for example, oily solutions, alcoholic and isopropanolic solutions (*e.g*., solutions of 2-octyl dodecanol or oleyl alcohol), solutions of esters of monocarboxylic acids (*e.g*., isopropyl myristate, isopropyl palmitate, lauric acid oxalic ester, oleic acid oleyl ester, oleic acid decyl ester, hexyl laurate, oleyl oleate, decyl oleate, and caproic acid esters of saturated fatty alcohols having a carbon chain of 12 to 18 carbons), solutions of esters of dicarboxylic acids (*e.g*., dibutyl phthalate, diisopropyl isophthalate, adipic acid diisopropyl ester, and di-n-butyl adipate), or solutions of esters of aliphatic acids (*e.g*., glycols). When the formulation comprises a spreading agent, it also may be advantageous to include a dispersant, such as, for example, pyrrolidin-2-one, N-alkylpyrrolidin-2-one, acetone, polyethylene glycol or an ether or ester thereof, propylene glycol, or synthetic triglycerides.

When formulated in, for example, an ointment, it is contemplated that the isoxazoline may be mixed with, for example, either a paraffinic or a water-miscible ointment base. When formulated in a cream, it is contemplated that the isoxazoline may be formulated with, for example, an oil-in-water cream base. In some instances, the aqueous phase of the cream base includes, for example at least about 30% (w/w) of a polyhydric alcohol, such as propylene glycol, butane-1,3-diol, mannitol, sorbitol, glycerol, polyethylene glycol, or a mixture thereof.

Injectable preparations may be formulated according to, for example, the known art using suitable solvents, solubilizing agents, protecting agents, dispersing agents, wetting agents, and/or suspending agents. Contemplated carrier materials include, for example, water, ethanol, butanol, benzyl alcohol, glycerin, 1,3-butanediol, Ringer's solution, isotonic sodium chloride solution, bland fixed oils (*e.g*., synthetic mono- or diglycerides), vegetable oil (*e.g*., corn oil), dextrose, mannitol, fatty acids (*e.g*., oleic acid), dimethyl acetamide, surfactants (*e.g*., ionic and non-ionic detergents), N-methylpyrrolidone, propylene glycol, and/or polyethylene glycols (*e.g*., PEG 400). Contemplated solubilizing agents include, for example, polyvinyl pyrrolidone, polyoxyethylated castor oil, polyoxyethylated sorbitan ester, and the like. Contemplated protecting agents include, for example, benzyl alcohol, trichlorobutanol, *p*-hydroxybenzoic acid ester, n-butanol, and the like.

In some embodiments, a parenteral formulation is, for example, prepared from sterile powders or granules having one or more of the carriers materials discussed above for other formulations. The isoxazoline is, for example, dissolved or suspended in a liquid comprising water, polyethylene glycol, propylene glycol, ethanol, corn oil, cottonseed oil, peanut oil, sesame oil, benzyl alcohol, sodium chloride, and/or various buffers. The pH generally may be adjusted, if necessary, with a suitable acid, base, or buffer.

For rectal administration, a suppository may be used. The suppository may be prepared by, for example, mixing a isoxazoline with a suitable non-irritating excipient that is solid at ordinary temperatures, but liquid at the rectal temperature, and will, therefore, melt in the rectum to release the drug. Contemplated excipients include, for example, such as cocoa butter; synthetic mono-, di-, or triglycerides; fatty acids; and/or polyethylene glycols.

Other inert ingredients may generally be added to the composition as desired. To illustrate, it is contemplated that these may include, for example, lactose, mannitol, sorbitol, calcium carbonate, sodium carbonate, tribasic calcium phosphate, dibasic calcium phosphate, sodium phosphate, kaolin, compressible sugar, starch, calcium sulfate, dextro or microcrystalline cellulose, colloidal silicon dioxide, starch, sodium starch glycolate, crospovidone, microcrystalline cellulose, tragacanth, hydroxypropylcellulose, pregelatinized starch, povidone, ethylcellulose, hydroxypropylcellulose, hydroxypropylmethylcellulose, and methylcellulose.

A general discussion regarding formulation of drugs and various excipients may be found in, for example, Gennaro, A.R., et al., eds., Remington: The Science and Practice of Pharmacy (Lippincott Williams & Wilkins, 20th Ed., 2000). Another general discussion regarding formulation of drugs and various excipients may be found in, for example, Liberman, H. A., et al., eds., Pharmaceutical Dosage Forms (Marcel Decker, New York, N.Y., 1980).

The concentration of the isoxazoline of **Formula (I)** (or any salt of the isoxazoline, solvate of the isoxazoline or salt, or prodrug of the isoxazoline) in the composition may vary widely depending on, for example, the mode of administration. In general, the concentration is from about 1 to about 70% (by weight). In some such embodiments, for example, the concentration is from about 1 to about 50% (by weight), or from about 10 to about 50% (by weight). In other embodiments, the concentration is from about 35 to about 65% (by weight), from about 40 to about 60% (by weight), from about 45 to about 55% (by weight), or about 50% (by weight).

### V. Examples of contemplated combination therapies

Also disclosed are methods wherein a isoxazoline is the sole active ingredient administered to the recipient animal. It is contemplated, however, that the methods also encompass combination therapies wherein a isoxazoline is administered in combination with one or more other active ingredients. The other active ingredient(s) may be, for example, one or more other isoxazolines. Alternatively (or additionally), the other active ingredient(s) may be one or more compounds that are not isoxazolines. The other active ingredient(s) may target the same and/or different pathogens and conditions.

Contemplated active ingredient(s) that may be administered in combination with the isoxazoline include, for example, anthelmintics, insecticides and acaricides, insect growth regulators and juvenile hormone analogues, anti-inflammatories, anti-infectives, hormones, dermatological preparations (*e.g*., antiseptics and disinfectants), and immunobiologicals (*e.g*., vaccines and antisera) for disease prevention.

Anthelmintics include, for example, avermectins (*e.g*., ivermectin, moxidectin, and milbemycin), benzimidazoles (*e.g*., fenbendazole, albendazole, and triclabendazole), salicylanilides (*e.g*., closantel and oxyclozanide), substituted phenols (*e.g.,* nitroxynil), pyrimidines (*e.g*., pyrantel), imidazothiazoles (*e.g*., levamisole), cyclooctadepsipeptide (*e.g.,* Emodepside), and tetrahydropyrimidines (*e.g*., praziquantel). Anthelmintics also include, for example, amino acetonitrile derivatives, such as, for example, those discussed in Kaminsky, R., et al., "A new class of anthelmintics effective against drug-resistant nematodes," Nature, vol. 452, pp. 176-180 (March 13, 2008); and Int'l Patent Publ. Nos. WO2006/050887 and WO2005/044784.

In some embodiments, the isoxazoline is administered in combination with (and, in some instances, in the same composition with) one or more macrocyclic lactone endectocidal parasiticides. These parasiticides tend to be useful against, for example, a broad spectrum of endoparasites and ectoparasites in mammals.

One particularly contemplated macrocyclic lactone parasiticide is ivermectin. Ivermectin is a semi-synthetic derivative of avermectin, and is generally produced as a mixture of at least 80% 22,23-dihydroavermectin B1ₐ and less than 20% 22,23-dihydroavermectin B1_{b}. Ivermectin is disclosed in US Patent 4,199,569. Ivermectin has been used as an antiparasitic agent to treat various parasitic diseases since the mid-1980's.

Other macrocyclic lactone parasiticides include, for example:
A. **Abamectin.** This compound is, for example, identified as avermectin B1ₐ/B1_{b} in U.S. Patent 4,310,519. Abamectin contains at least 80% of avermectin B1ₐ, and not more than 20% of avermectin B1_{b}.
B. **Doramectin.** This compound is known as 25-cyclohexyl-avermectin B₁. Its structure and preparation are discussed in, for example, US Patent 5,089,480.
C. **Moxidectin.** This compound is discussed in, for example, US Patent. 4,916,154.
D. **Selamectin.** This compound also is known as 25-cyclohexyl-25-de(1-methylpropyl)-5-deoxy-22, 23-dihydro-5-(hydroxyimino)-avermectin B1 monosaccharide.
E. **Milbemycin.** This compound also is known as B41. It is isolated from the fermentation broth of a Milbemycin-producing strain of *Streptomyces.* The microorganism, fermentation conditions, and isolation procedures are discussed in, for example, US Patents 3,950,360 and 3,984,564.
F. **Emamectin.** This compound also is known as 4"-deoxy-4"-epi-methylaminoavermectin B₁. Its preparation is discussed in, for example, US Patent Nos. 5,288,710 and 5,399,717. It is a mixture of two homologues, 4"-deoxy-4"-epi-methylaminoavermectin B1ₐ, and 4"-deoxy-4"-epi-methylaminoavermectin B1_{b}. Salt of emamectin are commonly used. Nonlimiting examples of such salts are those discussed in US Patent 5,288,710, which include salts derived from benzoic acid, substituted benzoic acid, benzenesulfonic acid, citric acid, phosphoric acid, tartaric acid, and maleic acid. A particularly contemplated salt is emamectin benzoate.
G. **Eprinomectin.** This compound is known as 4"-epi-acetylamino-4"-deoxy-avermectin B₁. It was developed for use in all cattle classes and age groups. It was the first avermectin to generally show broad-spectrum activity against both endo-and ecto-parasites, while also leaving minimal residues in meat and milk. It generally has an additional advantage of being highly potent when delivered topically.

Insecticides and acaricides include, for example, acephate, acetamiprid, acetoprole, amitraz, amidoflumet, avermectin, azadirachtin, azinphos-methyl, bifenthrin, bifenazate, buprofezin, bistrifluron, buprofezin, carbofuran, cartap, chlorfenapyr, chlorfluazuron, chlorantraniliprole), chlorpyrifos, chlorpyrifos-methyl, chromafenozide, clothianidin, cyflumetofen, cyfluthiin, β-cyfluthrin, cyhalothrin, γ-cyhalothrin λ-cyhalothrin, cypermethrin, cyromazine, deltamethrin, diafenthiuron, diazinon, dieldrin, diflubenzuron, dimefluthrin, dimethoate, dinotefuran, diofenolan, emamectin, endosulfan, esfenvalerate, ethiprole, fenothiocarb, fenoxycarb, fenpropathrin, fenvalerate, fipronil, flonicamid, flubendiamide, flucythrinate, tau-fluvalinate, flufenerim, flufenoxuron, fonophos, halofenozide, hexaflumuron, hydramethylnon, imidacloprid, indoxacarb, isofenphos, lufenuron, malathion, metaflumizone, metaldehyde, methamidophos, methidathion, methomyl, methoprene, methoxychlor, metofluthrin, monocrotophos, methoxyfenozide, monocrotophos, nitenpyram, nithiazine, novaluron, noviflumuron, oxamyl, .parathion, parathiori-methyl, permethrin, phorate, phosalone, phosmet; phosphamidori, pirimicarb, profenofos, profluthrin, protrifenbute, pymetrozine, pyrafluprole, pyrethrin, pyridalyl, pyrifluquinazon, pyriprole, pyriproxyfen, rotenone, ryanodine, spinetoram, spinosad, spirodiclofen, spiromesifen, spirotetramat, sulprofos, tebufenozide, teflubenzuron, tefluthrin, terbufos, tetrachlorvinphos, thiacloprid, thiamethoxam, thiodicarb, thiosultap-sodium, tolfenpyrad, tralomethrin, triazamate, trichlorfon, and triflumuron. General references discussing antiparasitic agents, such as insecticides and acaricides, include, for example, The Pesticide Manual, 13th Edition, C. D. S. Tomlin, Ed., British Crop Protection Council, Farnham, Surrey, U.K. (2003).

In some contemplated embodiments, the isoxazoline is administered with pyridylmethylamine derivatives, such as, for example, pyridylmethylamine derivatives discussed in European Patent Appl. EP0539588 or Int'l Patent Appl. Publ. WO2007/115643.

In some contemplated embodiments, the isoxazoline is administered with nodulisporic acids and derivatives thereof, such as, for example, compounds discussed in US Patent 5,399,582; 5,945,317; 5,962,499; 5,834,260; 6,221,894; or 5,595,991; or Int'l Patent Appl. Publ. 1996/29073.

Insect growth regulators include, for example, agridyne, diofenolan, fenoxycarb, hydroprene, kinoprene, methoprene, pyriproxyfen, tetrahydroazadirachtin, chlorfluazuron, cyromazine, diflubenzuron, fluazuron, flucycloxuron, flufenoxuron, hexaflumuron, ifenuron, tebufenozide, and triflumuron. These compounds tend to provide both initial and sustained control of parasites at all stages of insect development, including eggs, on the animal subject, as well as within the environment of the animal subject.

Other antiparasitic compounds contemplated to be useful in combination therapies with the isoxazoline include, for example, imidazo[1,2-b] pyridazine compounds discussed in US Patent Appl. Publ. No. 2005-0182059; 1-(4-Mono and di-halomethylsulphonylphenyl)-2-acylamino-3-fluoropropanol compounds discussed US Patent 7,361,689; trifluoromethanesulfonanilide oxime ether compounds discussed in US Patent 7,312,248; n-[(phenyloxy)phenyl]-1,1,1-trifluoromethanesulfonamide and n-[(phenylsulfanyl)phenyl]-1,1,1-trifluoromethanesulfonamide compounds discussed in US Patent Appl. Publ. 2006-0281695; and 2-phenyl-3-(1H-pyrrol-2-yl)acrylonitrile compounds discussed in US Appl. Publ. 2006/0128779.

Anti-inflammatory agents include, for example, corticosteroids, which, in turn, include, for example, beclomethasone dipropionate, betamethasone diproprionate, betamethasone valerate, budesonide, ciclesonide, deflazacort, dexamethasone, fluocinolone acetonide, fluticasone, propionate, fluticasone furoate, loteprednol, etabonate, mometasone, and mometasone furoate, methylprednisolone, prednisolone, prednisone, rofleponide, and triamcinolone acetonide. Anti-inflammatory agents also include, for example, one or more non-steroidal anti-inflammatory drugs ("NSAIDs"). NSAIDs include, for example, salicylates, arylalkanoic acids, 2-arylpropionic acids (or "profens"), N-arylanthranilic acids, pyrazolidine derivatives, oxicams, COX-2 inhibitors, sulphonanilides, and licofelone. Anti-inflammatory ingredients also may include, for example, antihistamines. Antihistamines include, for example, H₁-receptor agonists, H₂-receptor agonists, H₃-receptor agonists, H₄-receptor agonists, mast cell stabilizers, and vitamin C.

In the contemplated combination therapies, the isoxazoline of **Formula (I)** may be administered before, simultaneously, and/or after the other active ingredient(s). In addition, the isoxazoline may be administered in the same composition as the other active ingredient(s) and/or in a separate compositions from the other active ingredient(s). Further, the isoxazoline and other active ingredient(s) may be administered via the same and/or different routes of administration.

When the isoxazoline is administered in a combination therapy, the weight ratio of the active ingredients may vary widely. Factors influencing this ratio include, for example, the particular isoxazoline; the identity of the other active ingredient(s) be administered in the combination therapy; the mode(s) of administration of the isoxazoline and other active ingredient(s); the target condition and pathogen; the type (*e.g.,* species and breed), age, size, sex, diet, activity, and condition of the intended recipient; and pharmacological considerations, such as the activity, efficacy, pharmacokinetic, and toxicology profiles of the isoxazoline and other active ingredient(s). In some contemplated embodiments, for example, the weight radio of the isoxazoline to the other active ingredient(s) is, for example, is from about 1:3000 to about 3000:1. In some such instances, the weight ratio is from about 1:300 to about 300:1. In other such instances, the weight ratio is from about 1:30 and about 30:1.

In addition to other active ingredients, it is contemplated that the isoxazoline may be administered with one or more other compounds that beneficially affects (*e.g*., enhances or prolongs) the activity (or other characteristic, such as safety) of the isoxazoline. For example, it is contemplated that the isoxazoline may be administered with one or more synergists, such as, for example, piperonyl butoxide (PBO) and triphenyl phosphate (TPP). Other synergists include, for example, N-(2-ethylhexyl)-8,9,10-trinorborn-5-ene-2,3-dicarboxamide (also known as "ENT 8184" or "MGK 264") and Verbutin (also known as "MB-599"). A discussion relating to insecticidal synergists may be found in, for example, The Pesticide Manual, 13th Edition, cited above.

### EXAMPLES

The following examples are merely illustrative, and not limiting to the remainder of this disclosure in any way. The compound numbers referenced in these examples refer to the compound numbers for the structures in the above detailed description and below claims.

### Example 1. Model animal studies for Compound 11-1.

The objective of these studies was to assess the efficacy of Compound **11-1** against various parasites. The parasites were:

| **Arthropoda** | **Species** | **Family** | **Common name** |
|---|---|---|---|
| Insecta | *Ctenocephalides felis* | Pulicidae | Cat flea |
| Insecta | *Cimex lectularius* | Cimicidae | Bed bug |
| Acari | *Ornithodoros moubata* | Argasidae | Chicken tick |
| Acari | *Rhipicephalus sanguineus* | Ixodidae | Brown dog tick |
| Acari | *Myocoptes musculinus* | Myocoptidae | Rodent fur mite |

### A. Efficacy against mites on mice

All mice used in the study had a present mite infestation with *M. musculinus* consisting of all stages of the parasite with at least a medium ("++") infestation rate. The mice were divided into groups of three, and treated topically with 100 ppm body weight, orally with 10 mg/kg body weight, or subcutaneously with 10 mg/kg body weight of Compound **11**-1, fipronil (positive control), or nothing (negative control). This treatment was then repeated 7 days later. The infestation rate per mouse ("+" = low infestation rate, "++" = medium infestation rate, "+++" = high infestation rate) was assessed repeatedly on days 1, 6, 9, 13, and 23. Efficacy was defined as the inhibition of the mite infestation rate on treated mice relative to the negative control group. The results are shown in **Table 2.**

**Table 2**

| **Results for Experiment Assessing *In Vivo* Efficacy of Compound 11-1 Against Mites on Mice** | | | | | | |
|---|---|---|---|---|---|---|
| | **Day 0** | **Day 1** | **Day 6** | **Day 9** | **Day 13** | **Day 23** |
| **Infestation after oral administration of Compound 11-1** | ++ to +++ | + to ++ (remaining mites slightly damaged) | ++ | 2 mice: none detected | none detected | none detected |
| | | | | 1 mouse: + (remaining mites damaged) | | |
| **Infestation after subcutaneous administration of Compound 11-1** | ++ to +++ | + to ++ (remaining mites slightly damaged) | ++ | none detected | none detected | none detected |
| **Infestation after topical administration of Compound 11-1** | ++ to +++ | + (remaining mites severely damaged) | 2 mice: none detected | none detected | none detected | none detected |
| | | | 1 mouse: + (remaining mites damaged) | | | |
| **Infestation after oral administration of fipronil** | ++ to +++ | +++ | ++ to +++ | ++ to +++ | + | + |
| **Infestation after subcutaneous administration of fipronil** | ++ to +++ | +++ | 2 mice: + to ++ | 2 mice: + to ++ | + | + to ++ |
| | | | 1 mouse: +++ | 1 mouse: +++ | | |
| **Infestation after topical administration of fipronil** | ++ to +++ | 2 mice: + to ++ | + to ++ | 2 mice: none detected | none detected | none detected |
| | | 1 mouse: +++ (all mites damaged) | | 1 mouse: + (remaining mites damaged) | | |
| **Infestation with negative control** | ++ to +++ | ++ to +++ | ++ to +++ | ++ to +++ | ++ to +++ | ++ to +++ |

No side effects were observed with Compound 11-1 or fipronil during the mite efficacy study.

### Example 7. Isolation of the R and S enantiomers of Compound 11-1.

Compound **11**-1 (260 mg) was dissolved in a 1:1 mixture of n-hexane/ethanol (13 ml) at 40°C. 80% of this solution was separated into aliquots of 400 µl on a semi-preparative liquid chromatographic system equipped with a Diacel Chiralpak^{®} AD-H column with 250 mm column length, 10 mm diameter, and 5 µm particle size. The mobile phase consisted of a 8:2 mixture of n-hexane/ethanol. A flow rate of 4 ml/min was used. The chiral fractions of both enantiomers were collected and evaporated in vacuum. The purity of the pooled fractions was controlled by analytical chiral chromatography using a Diacel Chiralpak^{®} AD-H column (250 x 4.6 mm, 5 µm) and UV detection at 254 nm. For both enantiomers, a purity of greater than 99% was determined. This technique afforded 88 mg of Compound 17-1 (the S-enantiomer), which had an optical rotation of [α]_{D}²³ +63.97° (ethanol, c = 2.97 mg/ml); and 80 mg of Compound 11-1R (the R-enantiomer), which had an optical rotation of [α]_{D}²³ -61.07° (ethanol, c = 3.93 mg/ml).

The words "comprise", "comprises", and "comprising" are to be interpreted inclusively rather than exclusively. This interpretation is intended to be the same as the interpretation that these words are given under United States patent law.

The term "pharmaceutically acceptable" is used adjectivally to mean that the modified noun is appropriate for use in a pharmaceutical product. When it is used, for example, to describe a salt, excipient, or solvate, it characterizes the salt, excipient, or solvate as being compatible with the other ingredients of the composition, and not deleterious to the intended recipient animal to the extent that the deleterious effect(s) outweighs the benefit(s) of the salt, excipient, or solvate.

## Claims

1. An isoxazoline compound, a salt of the isoxazoline, or a solvate of the isoxazoline or salt;
the isoxazoline corresponds in structure to **Formula(I)**: 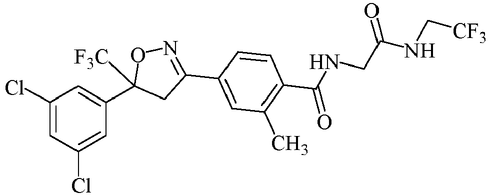 for use in effectively controlling an ectoparasite infestation of an animal, wherein the ectoparasites are mites and the isoxazoline compound is administered multiple times for a single treatment to the animal and such treatment controls such mites in the environment that is occupied by the animal.

2. The use of claim 1 wherein the doses are administered two or more days apart.

## Patentansprüche

1. Isoxazolinverbindung, Salz des Isoxazolins oder Solvat des Isoxazolins oder Salzes,
wobei die Struktur des Isoxazolins der **Formel (I)** entspricht: 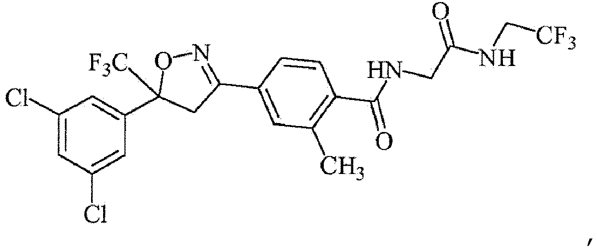 zur Verwendung bei der effektiven Bekämpfung eines Ektoparasitenbefalls eines Tieres, wobei es sich bei den Ektoparasiten um Milben handelt und die Isoxazolinverbindung für eine einzelne Behandlung dem Tier mehrmals verabreicht wird und durch eine solche Behandlung solche Milben in dem Umfeld, das von dem Tier genutzt wird, bekämpft werden.

2. Verwendung nach Anspruch 1, wobei die Dosen im Abstand von zwei oder mehr Tagen verabreicht werden.

## Revendications

1. Composé d'isoxazoline, sel de l'isoxazoline, ou solvate de l'isoxazoline ou du sel ; l'isoxazoline correspondant en structure à la formule (I) : 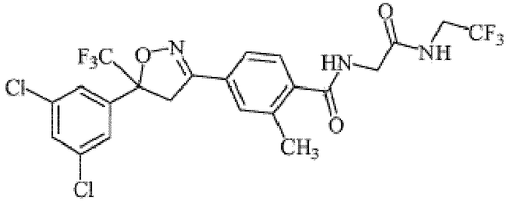 pour une utilisation dans la lutte de manière efficace contre une infestation d'un animal par un ectoparasite, les ectoparasites étant des mites et le composé d'isoxazoline étant administré plusieurs fois pour un traitement unique de l'animal et un tel traitement luttant contre les mites dans l'environnement qui est occupé par l'animal.

2. Utilisation selon la revendication 1, les doses étant administrées à deux ou plusieurs jours d'intervalle.
